# EUROPEAN PATENT SPECIFICATION

(11) **EP 3 466 478 B1**
(45) Date of publication and mention of the grant of the patent: **26.08.2020**
(21) Application number: 17810343.8
(22) Date of filing: 07.06.2017
(51) Int. Cl.: A61K 9/00, A61M 37/00, A61K 47/36, A61K 38/00, A61K 39/00, A61K 39/39, A61L 31/04

(54) **MICRONEEDLE ARRAY**
MIKRONADELARRAY
RÉSEAU DE MICRO-AIGUILLES

(30) Priority: 07.06.2016 JP 2016113449
(43) Date of publication of application: 10.04.2019
(73) Proprietor: FUJIFILM Corporation, Tokyo 106-8620 (JP)
(72) Inventor: KABATA, Koki, Ashigarakami-gun Kanagawa 258-8577 (JP); KUSANO, Takayuki, Ashigarakami-gun Kanagawa 258-8577 (JP)
(74) Representative: Klunker IP Patentanwälte PartG mbB
(86) International application number: PCT/JP2017/021117
(87) International publication number: WO 2017/213169

(56) References cited:
- EP-A1- 2 921 203
- EP-A2- 1 187 653
- JP-A- 2003 093 521
- JP-A- 2010 184 102
- JP-A- 2016 067 681
- JP-A- 2016 112 169

## Description

### Field of the Invention

The present invention relates to a microneedle array which includes a sheet and a plurality of needles.

### Related Art

As a method of administering a drug to the surface of an organism such as the skin or the mucous membrane, a method of adhering a liquid substance or a powdery substance to the surface of an organism may be exemplified. Further, in biopharmaceuticals which have been attracting attention recently, since it is extremely difficult to pass a drug through a barrier layer through infiltration, a method of administering a drug through an injection is selected. Further, as a method of administering a drug for the purpose of administering an appropriate amount of drug and achieving sufficient drug efficacy, a method of administering a drug using a microneedle array including microneedles containing a drug and having a high aspect ratio has been attracting attention. According to this microneedle array, a drug can be injected into the skin without pain by allowing microneedles to penetrate into a homy barrier layer. As an example of a microneedle array, a self-dissolving microneedle array that uses a substance having solubility in vivo as a base material has been reported. In the self-dissolving microneedle array, a drug can be intradermally administered by allowing the base material to hold a drug and allowing the base material to be self-dissolved during insertion of microneedles into the skin.

Patent Document 1 describes a microneedle chip which includes a support and a needle array region formed by providing a plurality of needles on a surface of this support, in which the support has a curved surface shape curved from the rear surface thereof toward the surface on which the needle array region is formed.

Patent Document 2 describes a needle-like body assembly chip formed by a plurality of fine needle-like bodies standing closely on a chip substrate, in which the height of needle-like bodies positioned in a central portion in a direction perpendicular to the surface of the chip substrate is set to be higher than the height of needle-like bodies positioned in an outer peripheral portion, at least needle-like bodies positioned in an outermost peripheral portion have a flat tip, and needle-like bodies positioned inside the outermost peripheral portion have a pointed end with an acute angle.

### Prior Art Documents

### Patent Documents

Patent Document 1: JP2013-158601A
Patent Document 2: JP2009-061144A
EP 2 921 203 A1 and EP 1 187 653 A2 each disclose a microneedle array comprising a sheet and a plurality of needles on an upper surface of the sheet.

### SUMMARY OF THE INVENTION

In a case where the method of adhering a drug which is a liquid substance or a powdery substance to the surface of an organism is used, since the region to which the drug adheres is limited to the surface of the skin, the adhered drug is occasionally removed due to perspiration or contact with foreign matter. Therefore, it is difficult to administer an appropriate amount of drug. Further, according to such a method of infiltrating a drug by means of diffusion of a drug, since the infiltration of the drug is inhibited by a horny barrier layer, it is difficult to obtain sufficient drug efficacy. In addition, administration of the drug through an injection is associated with pain or infection risk because the injection needs to be performed by medical workers.

A method of injecting a drug into the skin using a microneedle array has been attracting attention recently as a replacement of the method of administering a drug through an injection. The microneedle array punctures the target skin by being pushed with a puncture instrument (also referred to as an applicator) or a finger. The puncture is performed using constant energy that enables the puncture, but the sheet is required to withstand the puncture impact without being broken in order to puncture the skin with the microneedle with high reproducibility. The sheet can withstand the puncture impact by increasing the thickness of the sheet of the microneedle array, but it is preferable the amount of the polymer material, serving as a base material, to be used is small and the drying time in a production step is short because the microneedle array is required to be produced at a lower cost for mass production. Further, in order to puncture the microneedle array with high reproducibility into the skin of a human, it is more preferable that the microneedle array has flexibility to follow the curvature of the skin and important to achieve both of the strength and the flexibility. However, since the water-soluble polymer material having solubility in vivo frequently has a brittle property, desired effects cannot be obtained by only simply reducing the film thickness.

In the microneedle array described in Patent Documents 1 and 2, there is no description of film thickness distribution (in other words, the relationship between the film thickness of the end portion and the film thickness of the central portion) of the support or the chip substrate. Therefore, there is a concern that the sheet is damaged during the puncture because the microneedle array cannot withstand the impact of the puncture.

An object of the present invention is to provide a microneedle array having strength of withstanding the impact at the time of puncture.

As the result of intensive examination conducted by the present inventors in order to solve the above-described problems, it was found that a microneedle array having strength of withstanding the impact at the time of puncture can be provided by making the end portion thinner than the central portion as the film thickness distribution of the sheet. The present invention has been completed based on these findings.

In other words, according to the present invention, provided are the following embodiments.
[1] A microneedle array comprising: a sheet; and a plurality of needles which are present on an upper surface of the sheet, in which the needles contain a water-soluble polymer, the sheet contains a water-soluble polymer, the sheet has a central portion, which is a region where the plurality of needles are formed, and an end portion, and an average film thickness of the end portion is smaller than an average film thickness of the central portion. The sheet has a curved surface shape, and an absolute value of a deformation height of the curved surface is in a range of 1 µm to 300 µm.
[2] The microneedle array according to [1], in which the average film thickness of the end portion is 0.3 to 0.9 times the average film thickness of the central portion.
[3] The microneedle array according to [1] or [2], in which the average film thickness of the central portion is in a range of 100 µm to 300 µm.
[4] The microneedle array according to any one of [1] to [3], in which the needles contain a drug.
[5] The microneedle array according to any one of [1] to [4], in which a drug is one or more selected from a peptide hormone, a vaccine, and an adjuvant.
[6] The microneedle array according to any one of [1] to [5], in which the water-soluble polymer contained in the sheet contains dextran or hydroxyethyl starch.
[7] The microneedle array according to any one of [1] to [6], in which the water-soluble polymer of a needle base and the water-soluble polymer of the sheet are the same as each other.

The microneedle array of the present invention has strength of withstanding the impact at the time of puncture.

### BRIEF DESCRIPTION OF THE DRAWINGS

Fig. 1 is a view describing a needle tip and a needle base.
Fig. 2A is a perspective view illustrating a conical microneedle, Fig. 2B is a perspective view illustrating a pyramid-like microneedle, and Fig. 2C is a cross-sectional view illustrating a conical and pyramid-like microneedle.
Fig. 3 is a perspective view illustrating a microneedle in another shape.
Fig. 4 is a perspective view illustrating a microneedle in another shape.
Fig. 5 is a cross-sectional view of the microneedles illustrated in Figs. 3 and 4.
Fig. 6 is a perspective view illustrating a microneedle in another shape.
Fig. 7 is a perspective view illustrating a microneedle in another shape.
Fig. 8 is a cross-sectional view of the microneedles illustrated in Figs. 6 and 7.
Fig. 9 is a cross-sectional view of a microneedle in another shape in which the inclination (angle) of a side surface of a needle is continuously changed.
Fig. 10 illustrates a microneedle array including needles which are two-dimensionally arranged on a sheet.
Figs. 11A to 11C are step views illustrating a method of producing a mold.
Fig. 12 is an enlarged view of a mold.
Fig. 13 is a cross-sectional view illustrating a mold in another shape.
Figs. 14A to 14C are schematic views illustrating a step of filling the mold with a polymer-dissolved solution containing a drug.
Fig. 15 is a perspective view illustrating the tip of a nozzle.
Fig. 16 is a partially enlarged view of the tip of the nozzle and the mold during filling.
Fig. 17 is a partially enlarged view of the tip of the nozzle and the mold during movement.
Fig. 18 is a schematic configuration view illustrating a filling device.
Figs. 19A to 19D are views describing a step of forming a microneedle array.
Figs. 20A to 20C are views describing a step of forming a microneedle array.
Fig. 21 is a view describing a peeling step.
Fig. 22 is a view describing another peeling step.
Figs. 23A and 23B are views describing a microneedle array.
Fig. 24 illustrates an example of a method of producing a microneedle array.
Figs. 25A to 25C illustrate the definition of the average film thickness of the end portion and a method of measuring the average film thickness thereof.
Figs. 26A and 26B illustrate the definition of the average film thickness of the central portion and a method of measuring the average film thickness thereof.
Fig. 27 illustrates the definition of the absolute value of a deformation height and a method of measuring the absolute value thereof.

### DESCRIPTION OF THE PREFERRED EMBODIMENTS

Hereinafter, embodiments of the present invention will be described in detail.

In the present specification, the expression "containing a drug" means that a drug having an amount enough to exhibit drug efficacy is contained in a case where the body surface is punctured. The expression "not containing a drug" means that a drug having an amount enough to exhibit drug efficacy is not contained, and the range of the amount of the drug covers from a case where the drug is not contained at all to a case where the amount thereof is not enough to exhibit the drug efficacy.

Further, the numerical ranges shown using "to" in the present specification indicate ranges including the numerical values described before and after "to" as the lower limits and the upper limits.

In the present specification, "%" indicates "% by mass" unless otherwise specified.

### [Configuration of microneedle array]

A microneedle array according to the present invention includes a sheet; and a plurality of needles which are present on the upper surface of the sheet, in which the needles contain a water-soluble polymer. The needles contain a water-soluble polymer, the sheet contains a water-soluble polymer, the sheet has a central portion, which is a region where the plurality of needles are formed, and an end portion, and the average film thickness of the end portion is smaller than the average film thickness of the central portion. The sheet has a curved surface shape and an absolute value of a deformation height of the curved surface is in a range of 1 µm to 300 µm.

In the microneedle array according to the embodiment of the present invention, the strength of withstanding the impact at the time of puncture can be obtained by employing the configuration in which the average film thickness of the end portion is smaller than the average film thickness of the central portion. The fact that the strength of withstanding the impact at the time of puncture can be achieved by employing the above-described configuration is an effect that cannot be expected at all from the past.

### <Average film thickness of end portions>

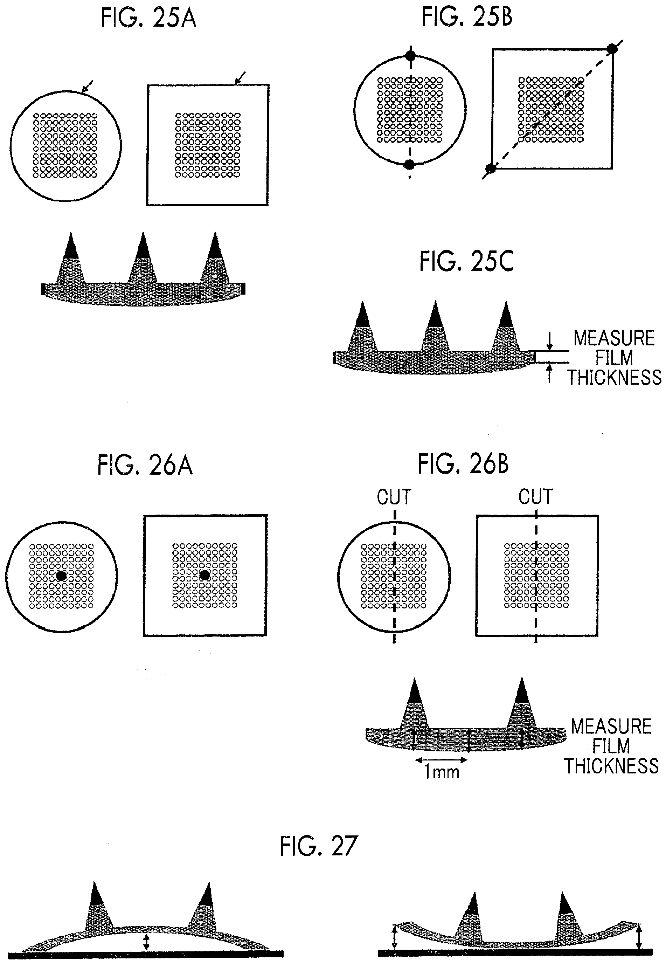

The definition of the average film thickness of the end portion and the method of measuring the average film thickness thereof will be described with reference to Figs. 25A to 25C.

Figs. 25A to 25C illustrate a case where the sheet has a circular shape and a case where the sheet has a square shape, but the shape of the sheet is not limited thereto.

The outermost peripheral portion of the sheet is defined as the end portion (Fig. 25A).

The average film thickness between two points of end portions on a straight line acquired by obtaining the end portions farthest from the center of the sheet and connecting the center of the sheet and the end portions farthest from the center thereof is defined as the average film thickness of the end portion (Fig. 25B).

According to a method of measuring the average film thickness of the end portion, the average film thickness of the end portion is acquired by observing the microneedle array at an angle of 90° horizontally and measuring the film thickness of the target end portion (Fig. 25C).

### <Average film thickness of central portion>

The definition of the average film thickness of the central portion and the method of measuring the average film thickness thereof will be described with reference to Figs. 26A and 26B.

The average film thickness of the central portion of the sheet is defined as the average film thickness of the central portion (Fig. 26A). The center of the sheet in the specification indicates the gravity point at the time of regarding the microneedle array as one sheet.

According to the method of measuring the average film thickness of the central portion, the microneedle array is cut so as to pass through the center of the sheet, the exposed cut cross section of the central portion is observed at an angle of 90° horizontally, measuring the film thickness (the film thickness of three sites in total) of the center and adjacent two sites separated from the center by a distance of 1 mm, and the average film thickness thereof is calculated as the average film thickness of the central portion (Fig. 26B).

### <Method of adjusting average film thickness of end portion and average film thickness of central portion>

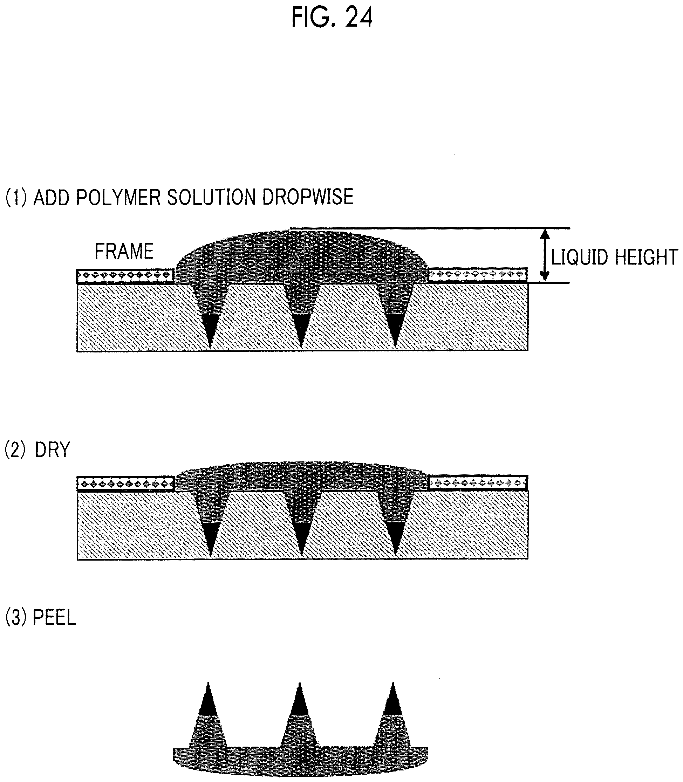

The method of producing the microneedle array according to the embodiment of the present invention is not particularly limited. For example, the microneedle array according to the embodiment of the present invention can be produced by preparing a polymer aqueous solution, filling a mold (a mold provided with a frame may be used as necessary) with the solution, drying the solution, and peeling the prepared microneedle array from the mold (Fig. 24).

Since the water-soluble polymer-dissolved solution is pinned to the frame, an end portion having a film thickness substantially the same as the thickness of the frame is formed. Accordingly, the average film thickness of the end portion can be adjusted by the thickness of the frame.

Further, the average film thickness of the central portion after molding can be adjusted by the liquid height and the concentration of the water-soluble polymer-dissolved solution.

For example, in a case where a water-soluble polymer-dissolved solution having a liquid height of approximately 1000 µm and a concentration of 40% (w/v) is added dropwise to a frame having a thickness of 0.2 mm and a diameter of 15 mm, a sheet which has a diameter of 15 mm and includes an end portion having an average film thickness of approximately 200 µm and a central portion having an average film thickness of approximately 400 µm is formed.

In addition, the method of producing a microneedle array will be described in detail.

In the present invention, the average film thickness of the end portion is smaller than the average film thickness of the central portion. The average film thickness of the end portion is typically in a range of 0.1 to 0.95 times the average film thickness of the central portion, and the average film thickness of the end portion is preferably in a range of 0.3 to 0.9 times the average film thickness of the central portion and more preferably in a range of 0.5 to 0.9 times the average film thickness of the central portion.

The average film thickness of the central portion is not particularly limited, but is preferably in a range of 100 µm to 300 µm.

### <Deformation height>

In the microneedle array according to the embodiment of the present invention, the sheet has a curved surface shape and the deformation height thereof decreases. The absolute value of the deformation height is in a range of 1 µm to 300 µm, preferably in a range of 1 µm to 250 µm, and more preferably in a range of 1 µm to 200 µm. In a low humidity environment in which the sheet is easily damaged, the damage to the sheet can be suppressed by decreasing (for example, setting the value to be in a range of 1 µm to 300 µm) the absolute value of the deformation height.

In a case where the microneedle array in a drying step illustrated in Fig. 24 is in a semi-dry state (for example, a paste state in which the shape of the solution changes even though the solution does not have fluidity) and the frame (preferably a stainless steel frame) and the mold are curved together, the frame and the mold are dried in a state of having an optional curved shape, and thus a sheet having an optional deformed shape is molded. Further, the bottom of the curved surface shape is likely to be a projection as the film thickness of the end portion decreases and the top thereof is likely to be a projection as the film thickness of the end portion increases. Therefore, the deformation height can be controlled by controlling the dropping amount and the thickness of the frame.

The curved surface shape indicates the above-described shape of the tendency of the "bottom becoming a projection" or the tendency of the "top becoming a projection".

The absolute value of the deformation height of the curved surface shape indicates the height from the target to a point positioned at the most distant place at the time of placing the microneedle array on the target to be punctured and performing observation at an angle of 90° horizontally (Fig. 27). The left view of Fig. 27 illustrates the case where the curved surface shape indicates the tendency of the "top becoming a projection" and the right view of Fig. 27 illustrates the case where the curved surface shape indicates the tendency of the "bottom becoming a projection". In the case where the curved surface shape indicates the tendency of the "top becoming a projection" (in a case where deformation of the central portion is large), the distance from the target to a point positioned at the most distant end portion is measured as the absolute value of the deformation height. In the case where the curved surface shape indicates the tendency of the "bottom becoming a projection" (in a case where deformation of the end portion is large), the average distance between two points obtained by measuring the distance from the target to a point positioned at the most distant place and the distance of the plane and the end portion corresponding to the diagonal thereof is defined as the absolute value of the deformation height.

### <Microneedle array>

In the present invention, plural means one or more.

The microneedle array according to the embodiment of the present invention includes at least a sheet and needles in order to efficiently administer the drug into the skin. In the microneedle array according to the embodiment of the present invention, it is preferable that the needles contain a drug.

The microneedle array according to the embodiment of the present invention is a device in which a plurality of needles are arranged in an array on the upper surface side of the sheet. It is preferable that the needles are arranged on the upper surface of the sheet at a predetermined density.

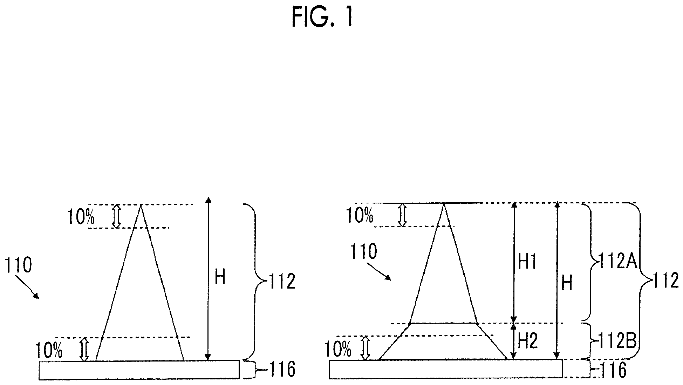

The sheet is a foundation for supporting needles and has a planar shape as the shape of the sheet 116 illustrated in Figs. 1 to 9. At this time, the upper surface of the sheet indicates the surface on which the plurality of needles are arranged in an array.

The area of the sheet is not particularly limited, but is preferably in a range of 0.005 to 1000 mm², more preferably in a range of 0.05 to 750 mm², and still more preferably in a range of 5 to 500 mm².

The sheet contains a water-soluble polymer. The sheet may be formed of a water-soluble polymer or may contain other additives (for example, disaccharides). Further, it is preferable that the sheet does not contain a drug.

The water-soluble polymer contained in the sheet is not particularly limited, and examples thereof include polysaccharides, polyvinylpyrrolidone, polyoxyethylene polyoxypropylene glycol, polyethylene glycol, polyvinyl alcohol, and protein (for example, gelatin). Examples of the polysaccharides include hyaluronic acid, sodium hyaluronate, pullulan, dextran, dextrin, chondroitin sulfate, sodium chondroitin sulfate, a cellulose derivative (for example, a water-soluble cellulose derivative obtained by partially modifying cellulose such as carboxymethyl cellulose, or hydroxypropyl cellulose), hydroxyethyl starch, and gum arabic. The above-described components may be used alone or in mixture of two or more kinds thereof. Among these, polysaccharides are preferable, hydroxyethyl starch, dextran, and hydroxypropyl cellulose are more preferable, and hydroxyethyl starch and dextran are particularly preferable. Among examples of the dextran, dextran 70 is particularly preferable.

Disaccharides may be added to the sheet and examples of the disaccharides include sucrose, lactulose, lactose, maltose, trehalose, and cellobiose. Among these, sucrose, maltose, and trehalose are particularly preferable.

The microneedle array is configured of a plurality of needles arranged in an array on the upper surface of the sheet. The needles have a projected structure with a tip, and the shape thereof is not limited to a needle shape having a sharp tip and may be a shape with a blunt tip.

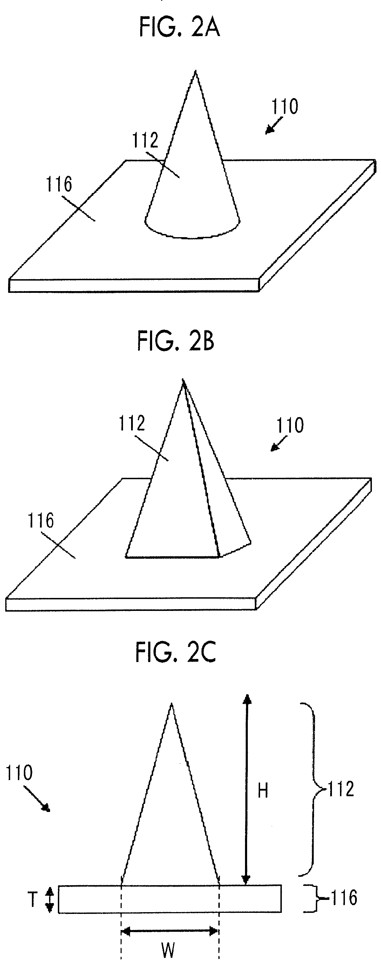

Examples of the shape of a needle include a conical shape, a polygonal pyramid shape (e.g. square pyramid shape), and a spindle shape. For example, a needle may have a shape of the needle 112 illustrated in any of Figs. 2 to 9, in which the entire shape of the needle may be a conical shape, a polygonal pyramid shape (e.g. square pyramid shape), or a shape of a structure in which the inclination (angle) of the side surface of the needle is continuously changed. Further, a needle may have a multilayer structure with two or more layers, in which the inclination (angle) of the side surface of the needle is discontinuously changed.

In a case where the microneedle array according to the embodiment of the present invention is applied to the skin, it is preferable that the needles are inserted into the skin and the upper surface or a part of the sheet is brought into contact with the skin.

The height (length) of a needle indicates the length of a perpendicular line drawn from the tip of the needle to the sheet. The height (length) of a needle is not particularly limited, but is preferably in a range of 50 µm to 3000 µm, more preferably in a range of 100 µm to 2000 µm, and still more preferably in a range of 100 µm to 1500 µm. It is preferable that the length of a needle is 50 µm or greater because a drug can be percutaneously administered. Further, it is preferable that the length of a needle is 3000 µm or less because occurrence of pain resulting from the contact of needles with the nerve is prevented and bleeding can be avoided.

The number of needles to be arranged in one microneedle array is preferably in a range of 1 to 2000, more preferably in a range of 3 to 1000, and still more preferably in a range of 5 to 500. In a case where one microneedle array includes two needles, the interval between needles indicates the distance between feet of each perpendicular line drawn from the tip of a needle to the sheet. In a case where one microneedle array includes three or more needles, the interval between needles to be arranged indicates an average value obtained by acquiring the distance between a foot of a perpendicular line drawn from the tip of a needle to the sheet and a foot of a perpendicular line drawn from the tip of a needle closest to the needle to the sheet and averaging the values obtained from all needles. The interval between needles is preferably in a range of 0.1 mm to 10 mm, more preferably in a range of 0.2 mm to 5 mm, and still more preferably in a range of 0.3 mm to 3 mm.

The needles contain a water-soluble polymer. It is preferable that the needles contain a drug other than the water-soluble polymer.

It is preferable that the water-soluble polymer is a biosoluble substance such that a human body is not damaged even though needles remain in the skin.

Examples of the water-soluble polymer contained in the needles include polysaccharides, polyvinylpyrrolidone, polyoxyethylene polyoxypropylene glycol, polyethylene glycol, polyvinyl alcohol, and protein (for example, gelatin). Examples of the polysaccharides include hyaluronic acid, sodium hyaluronate, pullulan, dextran, dextrin, chondroitin sulfate, sodium chondroitin sulfate, a cellulose derivative (for example, a water-soluble cellulose derivative obtained by partially modifying cellulose such as carboxymethyl cellulose, or hydroxypropyl cellulose), hydroxyethyl starch, and gum arabic. The above-described components may be used alone or in mixture of two or more kinds thereof. Among these, polysaccharides are preferable, and hydroxyethyl starch, hydroxypropyl cellulose, and dextran are more preferable.

Disaccharides may be added to the needles and examples of the disaccharides include sucrose, lactulose, lactose, maltose, trehalose, and cellobiose. Among these, sucrose, maltose, and trehalose are preferable.

In the needles, each needle tip and each needle base may be formed of different water-soluble polymers. In other words, the needles may be formed of two or more kinds of water-soluble polymers.

The needle tip is a region including the tip of a needle and indicates a needle tip region having a height corresponding to 10% of the height of the entire needle, and the needle base indicates a needle region from the bonding surface between the sheet and the needle to the height corresponding to 10% of the height of the entire needle. The region corresponding to the needle tip and the region corresponding to the needle base are respectively illustrated in Fig. 1. In the left view of Fig. 1, a sheet 116 is provided with one needle 112. H represents the height of the needle 112. As illustrated in the right view of Fig. 1, the needle may be formed of a needle first layer 112A and a needle second layer 112B. In the right view of Fig. 1, HI represents the height of the needle first layer 112A, H2 represents the height of the needle second layer 112B, and H represents the height of the entire needle.

It is preferable that the water-soluble polymer in the needle base is the same as the water-soluble polymer in the sheet.

It is preferable that the water-soluble polymer in the needle tip is hydroxyethyl starch.

It is preferable that the water-soluble polymer in the needle base is one or more selected from hydroxyethyl starch and dextran.

In a case where hydroxyethyl starch is used in the present invention, the weight-average molecular weight thereof is not particularly limited, but is preferably in a range of 70000 to 130000.

In a case where dextran is used in the present invention, the weight-average molecular weight thereof is not particularly limited, but is preferably in a range of 20000 to 200000. As the dextran, for example, dextran 40 (having a weight-average molecular weight of approximately 40000) or dextran 70 (having a weight-average molecular weight of approximately 70000) can be used. Further, since dextran becomes more brittle as the molecular weight thereof decreases, the weight-average molecular weight thereof is particularly preferably in a range of 50000 to 100000.

The microneedle array according to the embodiment of the present invention may or may not contain a drug, but it is preferable that the microneedle array contains a drug. It is preferable that the needles of the microneedle array contain a drug.

The drug indicates a substance that affects a human body. It is preferable that the drug is selected from peptides (including e.g. peptide hormones) or derivatives thereof, proteins, a nucleic acid, polysaccharides, vaccines, adjuvants, a pharmaceutical compound belonging to a water-soluble low molecular compound, or cosmetic ingredients. The molecular weight of the drug is not particularly limited, but a drug having a molecular weight of 500 or greater is preferable in a case of proteins.

Examples of peptides or derivatives thereof and proteins include calcitonin, adrenocorticotropic hormone, parathyroid hormone (PTH), human PTH (1 → 34), insulin, exendin, secretin, oxytocin, angiotensin, β-endorphin, glucagon, vasopressin, somatostatin, gastrin, luteinizing hormone releasing hormone, enkephalin, neurotensin, atrial natriuretic peptide, growth hormone, growth hormone releasing hormone, bradykinin, substance P, dynorphin, thyroid stimulating hormone, prolactin, interferon, interleukin, granulocyte colony stimulating factor (G-CSF), glutathione peroxidase, superoxide dismutase, desmopressin, somatomedin, endothelin, and salts of these.

Examples of the vaccines include influenza antigen (influenza vaccine), hepatitis B virus surface antigen (HBs) antigen, hepatitis Be antigen (HBe antigen), Bacille de calmette et Guerin (BCG) antigen, measles antigen, rubella antigen, varicella antigen, yellow fever antigen, shingles antigen, rotavirus antigen, influenza bacilli b type (Hib) antigen, rabies antigen, cholera antigen, diphtheria antigen, pertussis antigen, tetanus antigen, inactivated polio antigen, Japanese encephalitis antigen, human papilloma antigen, and antigens obtained by mixing two to four types of these.

Examples of the adjuvants include aluminum salts such as aluminum phosphate, aluminum chloride, and aluminum hydroxide, emulsions such as MF59 (registered trademark) and AS03 (trade name), liposomes, plant-derived components, a nucleic acid, biopolymers, cytokine, peptides, proteins, and sugar chains.

Among these, as the drug, at least one selected from the group consisting of peptide hormones, vaccines, and adjuvants is preferable. Growth hormone is particularly preferable as peptide hormones.

In a case where the needles of the microneedle array according to the embodiment of the present invention contain a drug, the content of the drug in all needles is not particularly limited, but is preferably in a range of 1% to 60% by mass, more preferably in a range of 1% to 50% by mass, and particularly preferably in a range of 1% to 45% by mass with respect to the mass of the solid content of needles.

Hereinafter, preferred embodiments of the present invention will be described with reference to the accompanying drawings, but the present invention is not limited thereto.

Figs. 2 to 8 are partially enlarged views illustrating a microneedle 110 in the microneedle array. The microneedle array according to the embodiment of the present invention is configured by the plurality of needles 112 being formed on the surface of the sheet 116 (in Figs. 2 to 8, one needle 112 is shown on the sheet 116 and this is referred to as the microneedle 110).

The needle 112 has a conical shape in Fig. 2A and the needle 112 has a square pyramid shape in Fig. 2B. In Fig. 2C, H represents the height of the needle 112, W represents the diameter (width) of the needle 112, and T represents the height (thickness) of the sheet 116.

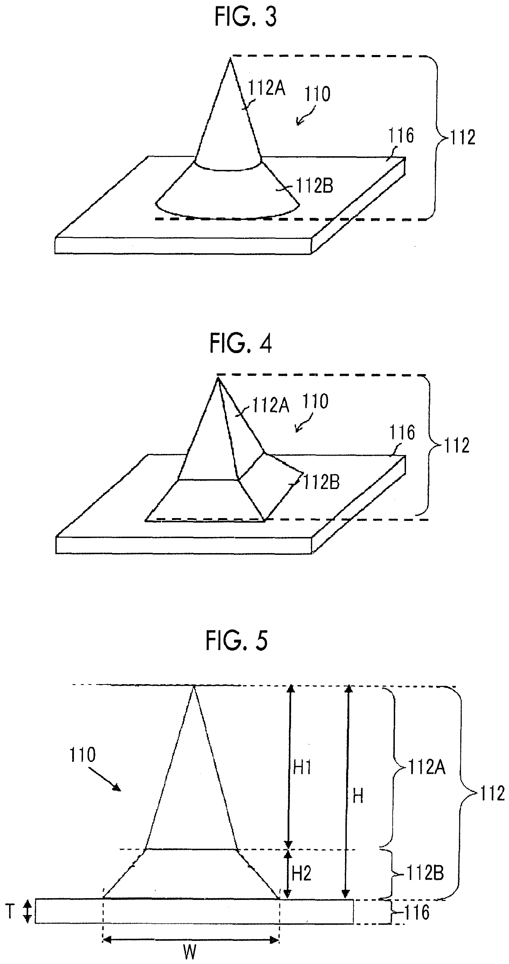

Figs. 3 and 4 respectively illustrate the microneedle 110 in another shape in which the needle 112 whose shape is discontinuously changed is formed on the surface of the sheet 116. In Fig. 3, the needle second layer 112B has a truncated conical shape and the needle first layer 112A has a conical shape. In Fig. 4, the needle second layer 112B has a truncated square pyramid shape and the needle first layer 112A has a square pyramid shape. However, the shape of the needle is not particularly limited.

Fig. 5 is a cross-sectional view illustrating the microneedles 110 illustrated in Figs. 3 and 4. In Fig. 5, H represents the height of the needle 112, W represents the diameter (width) of the base, and T represents the height (thickness) of the sheet 116.

It is preferable that the microneedle array according to the embodiment of the present invention has a shape of the microneedle 110 of Fig. 5 other than the shape of the microneedle 110 in Fig. 2C. With such a configuration, the volume of all needles becomes larger so that a greater amount of drug can be concentrated on the tip of a needle when the microneedle array is produced.

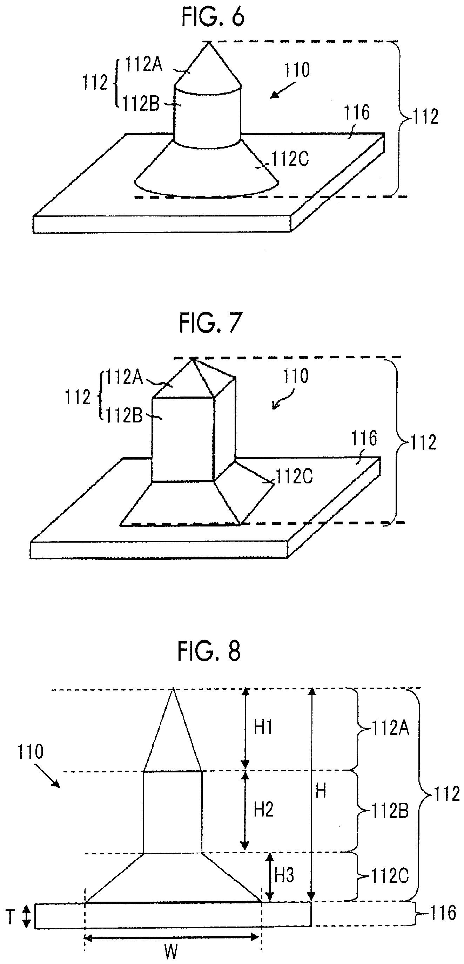

Figs. 6 and 7 illustrate microneedles 110 in different shapes.

The needle first layer 112A illustrated in Fig. 6 has a conical shape and the needle second layer 112B in Fig. 6 has a columnar shape. The needle first layer 112A illustrated in Fig. 7 has a square pyramid shape and the needle second layer 112B has a square columnar shape. However, the shape of a needle is not limited to these shapes. In Figs. 6 to 8, the reference numeral 112C represents a needle third layer.

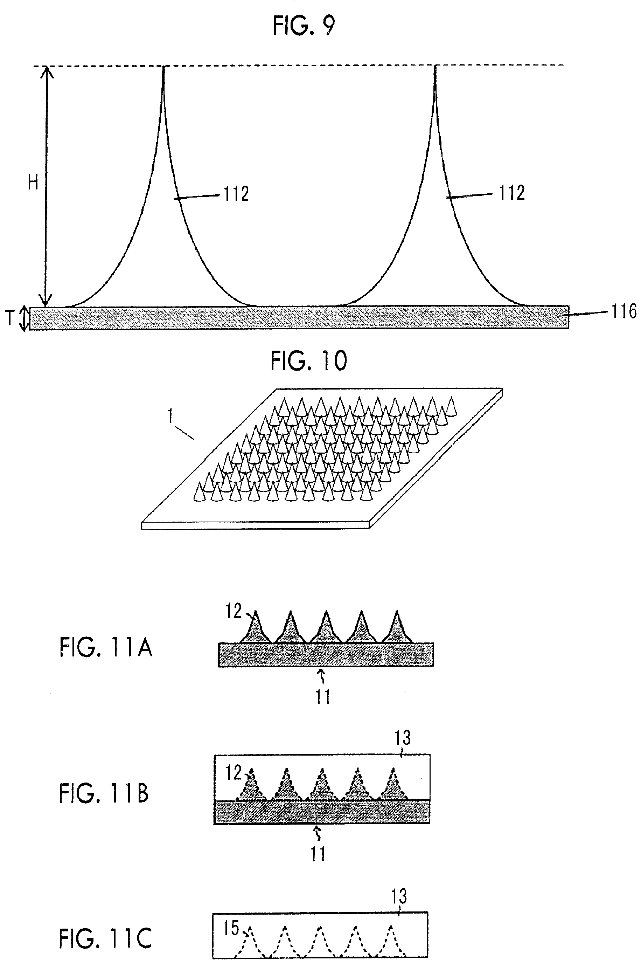

Fig. 8 is a cross-sectional view illustrating the microneedles 110 illustrated in Figs. 6 and 7. In Fig. 9, H represents the height of the needle 112, W represents the diameter (width) of the base, and T represents the height (thickness) of the sheet 116.

Fig. 9 is a cross-sectional view of a microneedle in another shape in which the inclination (angle) of the side surface of the needle 112 is continuously changed. In Fig. 9, H represents the height of the needle 112 and T represents the height (thickness) of the sheet 116.

In the microneedle array according to the embodiment of the present invention, as illustrated in Fig. 10, the needles are two-dimensionally arranged on the sheet. In the figure, the needles are arranged in a square shape, but the arrangement can be made in a circular shape or a diamond shape. Further, the position of the two-dimensional arrangement of needles is not limited.

It is preferable that needles are arranged at intervals of approximately 0.1 to 10 needles per 1 mm in a row. It is more preferable that the microneedle array has 1 to 10000 microneedles per 1 cm². In a case where the density of microneedles is set to 1 needle/cm² or greater, the microneedles can efficiently puncture the skin. In a case where the density of the microneedles is set to 10000 needles/cm² or less, the microneedle array can sufficiently puncture the skin. The density of needles is preferably in a range of 10 to 5000 needles/cm², more preferably in a range of 25 to 1000 needles/cm², and particularly preferably in a range of 25 to 400 needles/cm².

The microneedle array according to the embodiment of the present invention can be supplied in a sealed storage form together with a drying agent. As the drying agent, known drying agents (such as silica gel, calcined lime, calcium chloride, silica alumina, and a sheet-like drying agent) can be used.

### [Method of producing microneedle array]

The microneedle array according to the embodiment of the present invention can be produced by the following method in conformity with the method described in, for example, JP2013-153866A or WO2014/077242A.

### (Preparation of mold)

Figs. 11A to 11C are step views illustrating a method of preparing a mold (die). As illustrated in Fig. 11A, first, an original plate is prepared used to prepare the mold. There are two methods for preparing an original plate 11.

According to the first method, a Si substrate is coated with a photoresist, exposed, and then developed. Further, an array of shaped portions 12 having a conical shape (projection) is prepared on the surface of the original plate 11 by performing etching using e.g. reactive ion etching (RIE). In addition, in a case where the etching such as RIE is performed so as to form shaped portions having a conical shape on the surface of the original plate 11, the portions having a conical shape can be formed by performing etching in an oblique direction while the Si substrate rotates. According to the second method, an array of the shaped portions 12 having e.g. a square pyramid shape is formed on the surface of the original plate 11 by performing processing on a metal substrate such as Ni using a cutting tool such as a diamond bit.

Next, a mold is prepared. Specifically, a mold 13 is prepared using the original plate 11 as illustrated in Fig. 11B. As the method of preparing the mold, four methods are considered.

According to the first method, a silicone resin obtained by adding a curing agent to polydimethylsiloxane (PDMS, for example, SYLGARD 184 (registered trademark, manufactured by Dow Corning Toray Co., Ltd.)) is poured into the original plate 11, subjected to a heat treatment at 100°C, cured, and peeled from the original plate 11. According to the second method, an ultraviolet (UV) cured resin which is cured by being irradiated with ultraviolet rays is poured into the original plate 11, irradiated with ultraviolet rays in a nitrogen atmosphere, and peeled off from the original plate 11. According to the third method, a solution obtained by dissolving a plastic resin such as polystyrene or polymethyl methacrylate (PMMA) in an organic solvent is poured into the original plate 11 coated with a peeling agent, dried so that the organic solvent is volatilized, and cured, and then peeled off from the original plate 11. According to the fourth method, an inverted product is produced using Ni electroforming.

In this manner, the mold 13 formed by needle-like recesses 15, which have an inverted shape of the conical shape or the pyramid shape of the original plate 11, being two-dimensionally arranged is prepared. The mold 13 prepared in the above-described manner is illustrated in Fig. 11C.

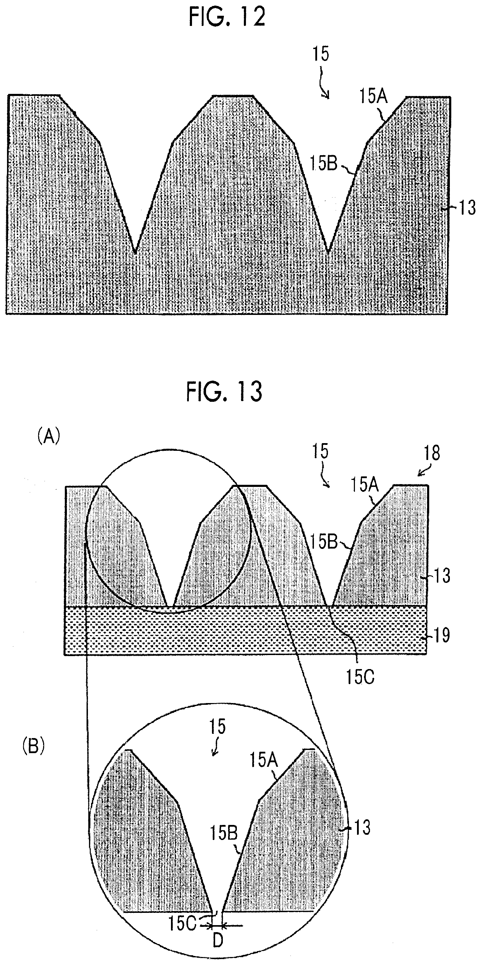

Fig. 12 illustrates another preferred embodiment of the mold 13. The needle-like recess 15 comprises a tapered inlet portion 15A which becomes narrower in a depth direction from the surface of the mold 13 and a tip recess 15B which becomes tapered in the depth direction. In a case where the inlet portion 15A has a tapered shape, the needle-like recess 15 is easily filled with the water-soluble polymer-dissolved solution.

Fig. 13 illustrates a more preferred embodiment of a mold complex 18 at the time of producing the microneedle array. The (A) portion of Fig. 13 illustrates the mold complex 18. The (B) portion of Fig. 13 is a partially enlarged view of a portion enclosed by a circle in the (A) portion.

As illustrated in the (A) portion of Fig. 13, the mold complex 18 comprises the mold 13 having an air vent hole 15C formed on the tip (bottom) of the needle-like recess 15; and an air permeating sheet 19 which is bonded to the rear surface of the mold 13 and is formed of a material that permeates a gas and does not permeate a liquid. The air vent hole 15C is formed as a through-hole penetrating the rear surface of the mold 13. Here, the rear surface of the mold 13 indicates the surface on a side on which the air vent hole 15C is formed. With this configuration, the tip of the needle-like recess 15 communicates with the air through the air vent hole 15C and the air permeating sheet 19.

In a case where such a mold complex 18 is used, only the air present in the needle-like recess 15 can be released from the needle-like recess 15 without permeation of the polymer-dissolved solution filling the needle-like recess 15. In this manner, the property of transferring the shape of the needle-like recess 15 to a polymer becomes excellent in the above-described manner and a sharper needle can be formed.

A diameter D (diameter) of the air vent hole 15C is preferably in a range of 1 to 50 µm. In a case where the diameter D of the air vent hole 15C is less than 1 µm, the air vent hole 15C cannot be sufficiently used as an air bend hole. Further, in a case where the diameter D of the air vent hole 15C is greater than 50 µm, the sharpness of the tip of a formed microneedle is damaged.

As the air permeating sheet 19 formed of a material that permeates a gas and does not permeate a liquid, for example, a gas permeating film (Poreflon (registered trademark), FP-010, manufactured by Sumitomo Electric Industries, Ltd.) can be suitably used.

As the material used for the mold 13, an elastic material or a metal material can be used. Among these, an elastic material is preferable and a material having a high gas permeability is more preferable. The oxygen permeability, which is a representative example of the gas permeability, is preferably 1 × 10⁻¹² (mL/s·m²·Pa) or greater and more preferably 1 × 10⁻¹⁰ (mL/s·m²·Pa) or greater. Further, 1 mL is 10⁻⁶ m³. In a case where the gas permeability is in the above-described range, the air present in a recess of the mold 13 can be released from the die and a microneedle array with less defects can be produced. Specific examples of such materials include materials obtained by melting or dissolving, in a solvent, a silicone resin (for example, SYLGARD 184 (registered trademark, manufactured by Dow Coming Toray Co., Ltd.) or KE-1310ST (product number, manufactured by Shin-Etsu Chemical Co., Ltd.)), a UV curable resin, or a plastic resin (for example, polystyrene or polymethyl methacrylate (PMMA)). Among these, a silicone rubber-based material is preferable since the material has durability to transfer resulting from repetitive pressure and has excellent peeling properties with respect to a material. Further, examples of the metal material include Ni, Cu, Cr, Mo, W, Ir, Tr, Fe, Co, MgO, Ti, Zr, Hf, V, Nb, Ta, α-aluminum oxide, zirconium oxide, stainless (for example, STAVAX (registered trademark) of Bohler-Uddeholm KK), and alloys thereof.

As the material of a frame, the same material as the material of the mold 13 can be used.

### (Water-soluble polymer-dissolved solution)

In the present invention, it is preferable to prepare a water-soluble polymer-dissolved solution containing a drug used to form at least a part of a needle and a water-soluble polymer-dissolved solution used to form the sheet.

The type of water-soluble polymer is as described in the present specification above.

Disaccharides may be mixed into the water-soluble polymer-dissolved solution, and the type of the disaccharides is as described in the present specification above.

The concentration of the water-soluble polymer in any of the water-soluble polymer-dissolved solutions varies depending on the type of the water-soluble polymer to be used, and is preferably in a range of 1 to 50% by mass. Further, a solvent used for dissolution may be a solvent other than water as long as the solvent has volatility, and methyl ethyl ketone (MEK) or an alcohol can be used as the solvent.

### (Formation of needle)

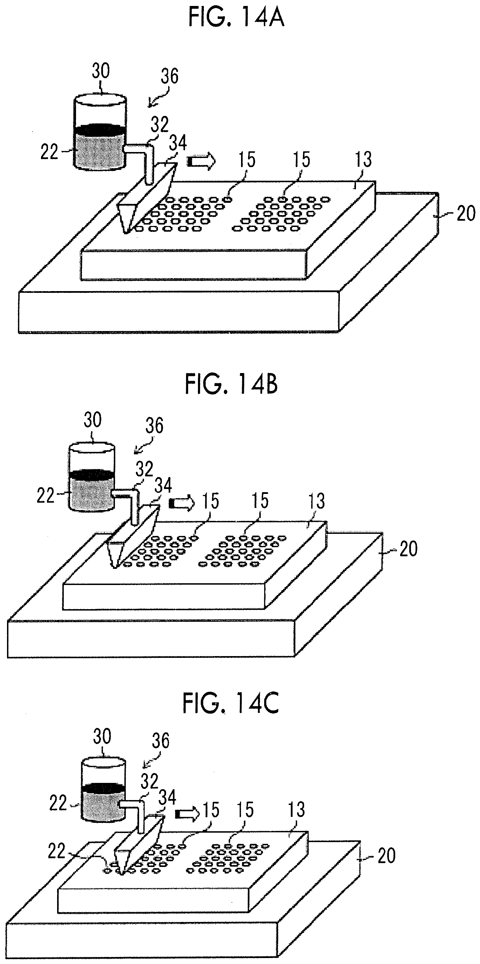

As illustrated in Fig. 14A, the mold 13 having needle-like recesses 15 which are two-dimensionally arranged is disposed on a base 20. In the mold 13, two sets of plural needle-like recesses 15 are formed such that 5 rows of needle-like recesses 15 and 5 columns of needle-like recesses 15 are two-dimensionally arranged. A liquid supply device 36 including a tank 30 which accommodates a water-soluble polymer-dissolved solution 22 containing a drug; a pipe 32 which is connected with the tank; and a nozzle 34 which is connected with the tip of the pipe 32 is prepared. Further, in the present example, the case where 5 rows of needle-like recesses 15 and 5 columns of needle-like recesses 15 are two-dimensionally arranged is exemplified, but the arrangement and the number of the needle-like recesses 15 is not limited to 5 rows × 5 columns. Further, the arrangement thereof is not limited to the arrangement in a square shape, and two-dimensional arrangement in a circular shape may be employed.

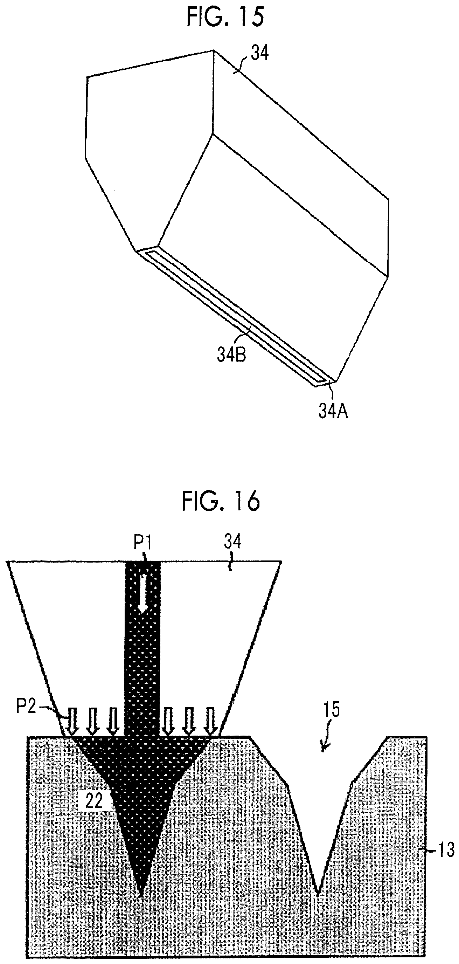

Fig. 15 is a perspective view schematically illustrating the tip portion of the nozzle. As illustrated in Fig. 15, the tip of the nozzle 34 comprises a lip portion 34A which is a flat surface and an opening portion 34B having a slit shape. For example, a plurality of needle-like recesses 15 forming one row can be concurrently filled with the water-soluble polymer-dissolved solution 22 containing a drug because of the opening portion 34B having a slit shape. The size (the length and the width) of the opening portion 34B can be suitably selected according to the number of needle-like recesses 15 to be filled with the water-soluble polymer-dissolved solution at the same time. In a case where the length of the opening portion 34B is set to be large, a larger amount of needle-like recesses 15 can be filled with the polymer-dissolved solution 22 containing a drug at the same time. In this manner, the productivity can be improved.

As the material used for the nozzle 34, an elastic material or a metal material can be used. Examples thereof include TEFLON (registered trademark), stainless steel (steel special use stainless (SUS)), and titanium.

As illustrated in Fig. 14B, the position of the opening portion 34B of the nozzle 34 is adjusted on the needle-like recesses 15. The lip portion 34A of the nozzle 34 is in contact with the surface of the mold 13. The water-soluble polymer-dissolved solution 22 containing a drug is supplied to the mold 13 from a liquid supply device 36, and the water-soluble polymer-dissolved solution 22 containing a drug fills the needle-like recesses 15 from the opening portion 34B of the nozzle 34. In the present embodiment, a plurality of needle-like recesses 15 forming one row can be concurrently filled with the water-soluble polymer-dissolved solution 22 containing a drug. However, the present invention is not limited thereto, and the needle-like recesses 15 can be filled with the water-soluble polymer-dissolved solution one by one.

In a case where the mold 13 is formed of a material having a gas permeability, the water-soluble polymer-dissolved solution 22 containing a drug can be suctioned by suctioning the solution from the rear surface of the mold 13, and the filling of the needle-like recesses 15 with the water-soluble polymer-dissolved solution 22 containing a drug can be promoted.

Next to the filling step with reference to Fig. 14B, as illustrated in Fig. 14C, the lip portion 34A of the nozzle 34 is brought into contact with the surface of the mold 13, the liquid supply device 36 is relatively moved in the length direction and the vertical direction of the opening portion 34B, and the nozzle 34 is moved to the needle-like recesses 15 which are not filled with the water-soluble polymer-dissolved solution 22 containing a drug. The position of the opening portion 34B of the nozzle 34 is adjusted on the needle-like recesses 15. In the present embodiment, the example of moving the nozzle 34 has been described, but the mold 13 may be moved.

Since the lip portion 34A of the nozzle 34 is brought into contact with the surface of the mold 13 and then the movement is made, the water-soluble polymer-dissolved solution 22 containing a drug, which remains on the surface other than the needle-like recesses 15 of the mold 13 can be collected by the nozzle 34. It is possible to prevent the water-soluble polymer-dissolved solution 22 containing a drug from remaining on the surface other than the needle-like recesses 15 of the mold 13.

In order to reduce the damage to the mold 13 and suppress deformation due to compression of the mold 13 as much as possible, it is preferable that the pressing pressure of the nozzle 34 against the mold 13 is set to be as small as possible during the movement. Further, in order to prevent the water-soluble polymer-dissolved solution 22 containing a drug from remaining on the surface other than the needle-like recesses 15 of the mold 13, it is preferable that at least one of the mold 13 or the nozzle 34 is formed of a flexible material which can be elastically deformed.

By repeating the filling step of Fig. 14B and the moving step of Fig. 14C, 5 rows and 5 columns of needle-like recesses 15 which are two-dimensionally arranged are filled with the water-soluble polymer-dissolved solution 22 containing a drug. In a case where 5 rows and 5 columns of needle-like recesses 15 which are two-dimensionally arranged are filled with the water-soluble polymer-dissolved solution 22 containing a drug, the liquid supply device 36 is moved to 5 rows and 5 columns of two-dimensionally arranged needle-like recesses 15 which are adjacent to the needle-like recesses filled with the solution and then the filling step of Fig. 14B and the moving step of Fig. 14C are repeated. The 5 rows and 5 columns of two-dimensionally arranged needle-like recesses 15 which are adjacent to the needle-like recesses filled with the solution are filled with the water-soluble polymer-dissolved solution 22 containing a drug.

For the above-described filling step and moving step, (1) embodiment in which the needle-like recesses 15 are filled with the water-soluble polymer-dissolved solution 22 containing a drug while the nozzle 34 is moved or (2) embodiment in which the nozzle 34 is temporarily stopped on the needle-like recesses 15 during the movement of the nozzle 34, the needle-like recesses 15 are filled with the water-soluble polymer-dissolved solution 22 containing a drug, and the nozzle 34 is moved again after the filling may be adopted. The lip portion 34A of the nozzle 34 is brought into contact with the surface of the mold 13 between the filling step and the moving step.

Fig. 16 is a partially enlarged view of the mold 13 and the tip of the nozzle 34 at the time of filling the needle-like recess 15 with the water-soluble polymer-dissolved solution 22 containing a drug. As illustrated in Fig. 16, the filling of the needle-like recess 15 with the water-soluble polymer-dissolved solution 22 containing a drug can be promoted by applying a pressing pressure P1 into the nozzle 34. Further, in a case where the needle-like recess 15 is filled with the water-soluble polymer-dissolved solution 22 containing a drug, it is preferable that a pressing force P2 for bringing the nozzle 34 into contact with the surface of the mold 13 is set to be greater than or equal to the pressing pressure P1 applied into the nozzle 34. In a case where the pressing force P2 is set to be greater than or equal to the pressing pressure PI, it is possible to suppress leaking of the water-soluble polymer-dissolved solution 22 containing a drug to the surface of the mold 13 from the needle-like recess 15.

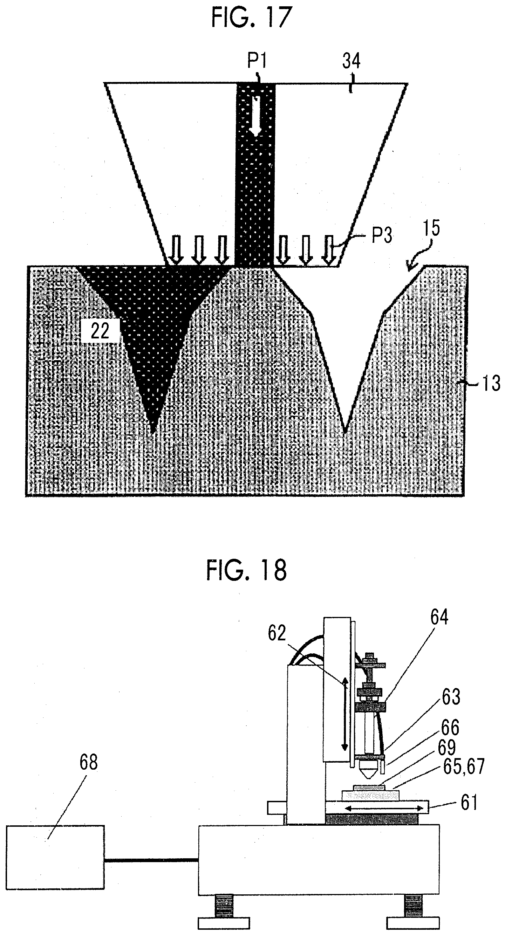

Fig. 17 is a partially enlarged view of the tip of the nozzle 34 and the mold 13 during the movement of the nozzle 34. In a case where the nozzle 34 is relatively moved with respect to the mold 13, it is preferable that a pressing force P3 of bringing the nozzle 34 into contact with the surface of the mold 13 is set to be smaller than the pressing force P2 of bringing the nozzle 34 into contact with the surface of the mold 13 during the filling. In a case where the pressing force P3 is set to be smaller than the pressing force P2, the damage to the mold 13 is reduced and the deformation of the mold 13 due to compression is suppressed.

It is preferable that the nozzle 34 is driven in the Z axis direction according to the surface shape of the mold 13 to control the pressing force and/or the pushing distance of the nozzle 34 to the mold 13.

Fig. 18 is a schematic configuration view illustrating a filling device which is capable of controlling the pressing force and/or the pushing distance. The filling device comprises an X-axis drive unit 61 and a Z-axis drive unit 62 which control relative position coordinates of a mold and a nozzle; a liquid supply device 64 (ultratrace determination dispenser SMP-III, manufactured by Musashi Engineering, Inc.) to which the nozzle 63 is attachable; a suction stand 65 which fixes a mold 69; a laser displacement meter 66 (HL-C201A, manufactured by Panasonic Corporation) which measures the shape of the mold surface; a load cell 67 (LCX-A-500N, manufactured by Kyowa Electronic Instruments Co., Ltd.) which measures the pressing pressure of the nozzle; and a control mechanism 68 which controls the Z-axis based on data of measured values of the surface shape and the pressing pressure.

In a case where the filling of the plurality of needle-like recesses 15 formed of 5 rows and 5 columns of needle-like recesses is completed, the nozzle 34 is moved to the plurality of needle-like recesses 15 formed of 5 rows and 5 columns of needle-like recesses adjacent to the needle-like recesses filled with the solution. In a case where the nozzle 34 is moved to the plurality of needle-like recesses 15 formed of 5 rows and 5 columns of needle-like recesses adjacent to the needle-like recesses filled with the solution at the time of liquid supply, it is preferable that the supply of the water-soluble polymer-dissolved solution 22 containing a drug is stopped. There is a distance between the needle-like recesses 15 in the fifth row and the needle-like recesses 15 in the next first row. In a case where the water-soluble polymer-dissolved solution 22 containing a drug is continuously supplied during the movement of the nozzle 34 between the rows, the liquid pressure inside of the nozzle 34 is extremely high in some cases. As the result, the water-soluble polymer-dissolved solution 22 containing a drug supplied from the nozzle 34 occasionally flows out of the needle-like recesses 15 of the mold 13. In order to suppress the flowing out of the solution, it is preferable that the liquid pressure inside the nozzle 34 is detected and the supply of the water-soluble polymer-dissolved solution 22 containing a drug is stopped in a case where it is determined that the liquid pressure is extremely high.

In the above, the method of supplying the water-soluble polymer-dissolved solution containing a drug using a dispenser that has a nozzle has been described, but bar coating, spin coating, or spray coating can be applied in addition to the coating with the dispenser.

In the present invention, it is preferable that the drying treatment is performed after the water-soluble polymer-dissolved solution containing a drug is supplied to the needle-like recesses.

Preferably, the microneedle array according to the embodiment of the present invention can be produced by performing a step of forming some needles by drying a mold for forming needles, filled with the first water-soluble polymer-dissolved solution; and a step of filling the upper surface of some needles formed in the above-described manner with the second water-soluble polymer-dissolved solution and drying the mold. It is preferable that a solution containing a drug is used as the first water-soluble polymer-dissolved solution.

### (Formation of sheet)

Several embodiments of a step of forming the sheet will be described.

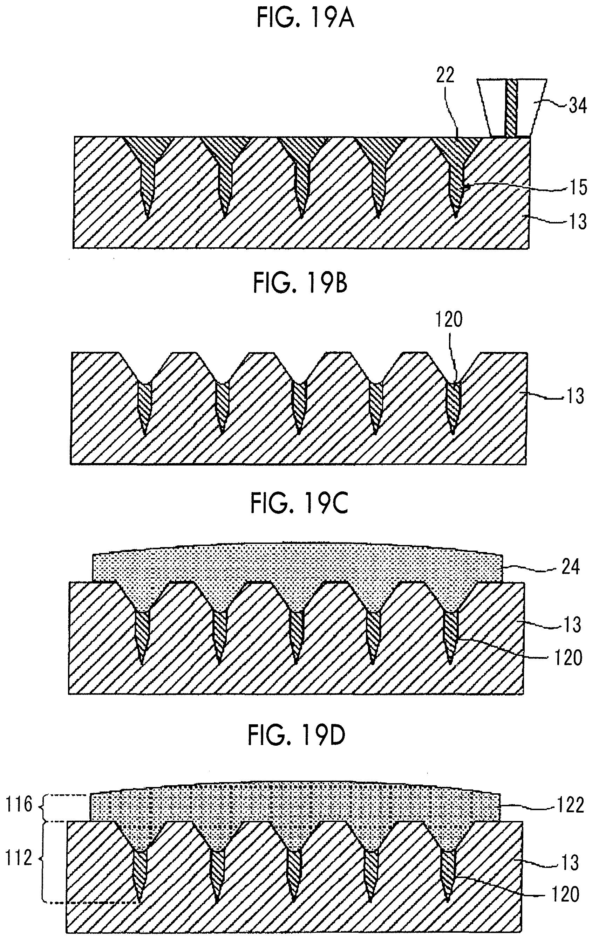

A first embodiment of a step of forming the sheet will be described with reference to Figs. 19A to 19D. The needle-like recesses 15 of the mold 13 are filled with the water-soluble polymer-dissolved solution 22 containing a drug from the nozzle 34. Next, a layer 120 in the needle-like recesses 15 is formed by drying and solidifying the water-soluble polymer-dissolved solution 22 containing a drug as illustrated in Fig. 19B. Subsequently, the mold 13 on which the layer 120 is formed is coated with the water-soluble polymer-dissolved solution 24 using a dispenser as illustrated in Fig. 19C. In addition to the coating with the dispenser, bar coating, spin coating, or spray coating can be applied. Since the layer 120 is solidified, it is possible to suppress the diffusion of the drug in the water-soluble polymer-dissolved solution 24 even in a case where the layer 120 contains a drug. Next, the microneedle array 1 including a plurality of needles 112 and the sheet 116 is formed by drying and solidifying the water-soluble polymer-dissolved solution 24 as illustrated in Fig. 19D.

In the first embodiment, in order to promote the filling of the needle-like recesses 15 with the water-soluble polymer-dissolved solution 22 and the water-soluble polymer-dissolved solution 24 containing a drug, it is preferable to apply a pressure from the surface of the mold 13 and perform suctioning from the rear surface of the mold 13 under reduced pressure.

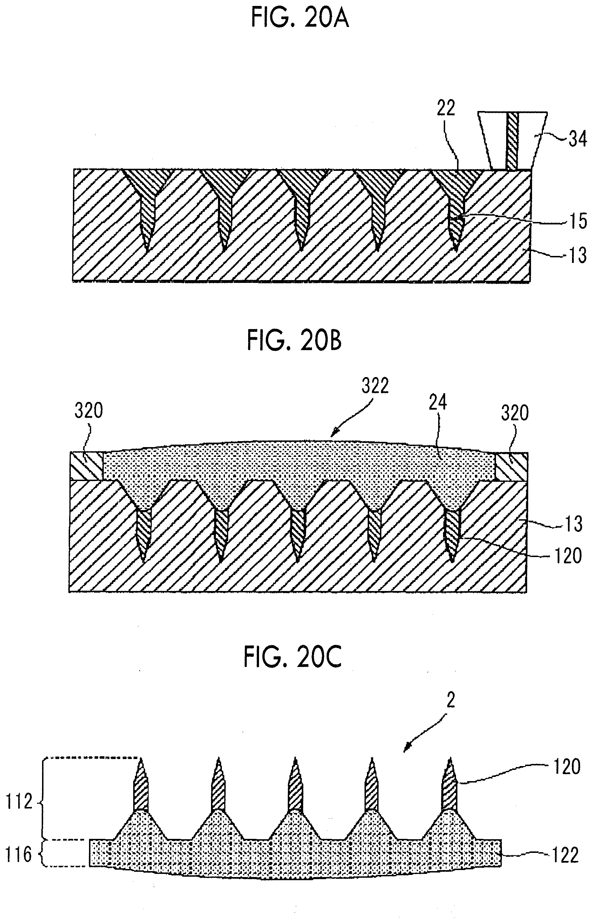

Subsequently, a second embodiment will be described with reference to Figs. 20A to 20C. The needle-like recesses 15 of the mold 13 are filled with the water-soluble polymer-dissolved solution 22 from the nozzle 34 as illustrated in Fig. 20A. Next, similar to Fig. 19B, the layer 120 is formed in the needle-like recesses 15 by drying and solidifying the water-soluble polymer-dissolved solution 22. Next, a frame 320 having an opening portion 322 is prepared, as illustrated in Fig. 20B, the needle-like recess of the mold 13 and the opening portion 322 are positioned, and the frame 320 is disposed on the surface (the side of the mold filled with the water-soluble polymer-dissolved solution 24) of the mold 13. In this state, the mold is filled with the water-soluble polymer-dissolved solution 24.

As illustrated in Fig. 20C, in a case where the water-soluble polymer-dissolved solution 24 filling the opening portion 322 of the frame 320 is dried, the water-soluble polymer-dissolved solution 24 is dried and contracted. Since the water-soluble polymer-dissolved solution 24 is in contact with the side surface of the frame 320, the portion contact in the side surface is not largely contracted.

As the method of drying the water-soluble polymer-dissolved solution 24, a step of volatilizing the solvent in the water-soluble polymer-dissolved solution may be exemplified. The method is not particularly limited, and a method of performing heating, blowing air, or decompression may be used. The drying treatment can be performed under the conditions of 1°C to 50°C for 1 to 72 hours. Examples of the method of blowing air include a method of blowing hot air at 0.1 to 10 m/sec. In a case where the water-soluble polymer-dissolved solution 22 contains a drug, it is preferable that the drying temperature is set to a temperature at which the drug in the water-soluble polymer-dissolved solution 22 is not thermally deteriorated.

### (Peeling)

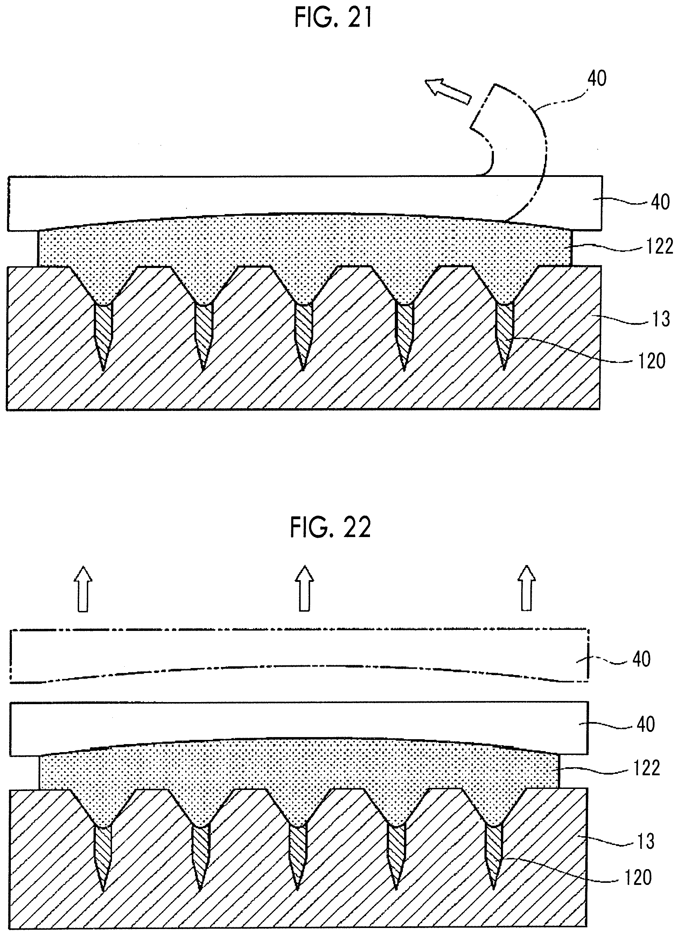

A method of peeling the microneedle array from the mold 13 is not particularly limited. It is preferable that needles are not bent or broken at the time of peeling. Specifically, a sheet-like base material 40 on which a pressure sensitive adhesive layer is formed is attached to the microneedle array and then the base material 40 can be peeled off from the end portion such that the base material 40 is turned over as illustrated in Fig. 21. However, the needles can be bent in a case where this method is used. Therefore, as illustrated in Fig. 22, a sucking disc (not illustrated) is disposed on the base material 40 on the microneedle array so that a method of vertically pulling the base material up while suctioning the base material with air can be applied. Further, a sucking disc is disposed on the rear surface of the sheet of the microneedle array without using the base material 40 and the sheet can be vertically pulled up while suctioning the sheet with air can be applied.

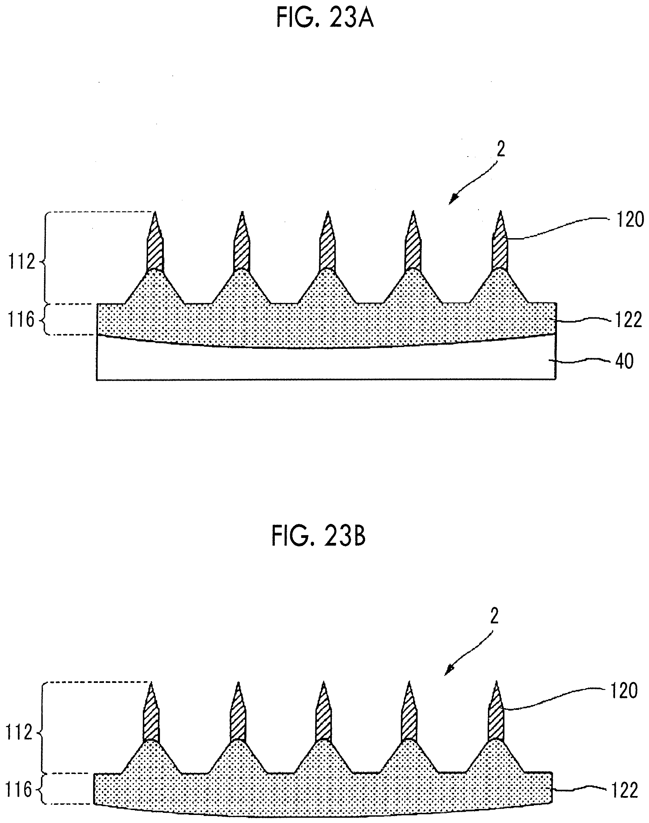

Figs. 23A and 23B illustrate the microneedle array 2 peeled from the mold 13.

The microneedle array 2 illustrated in Fig. 23A includes the base material 40, the needles 112 formed on the base material 40, and the sheet 116. At least the tip of the needle 112 has a conical shape or a polygonal pyramid shape, but the shape of the needle 112 is not limited thereto.

The microneedle array 2 illustrated in Fig. 23B is a microneedle array obtained by disposing a sucking disk on the rear surface of the sheet of the microneedle array without using the base material 40 and vertically pulling the sheet up while suctioning the sheet with air.

Hereinafter, the present invention will be described in detail with reference to examples. The materials, the amounts to be used, the ratios, the treatment contents, and the treatment procedures shown in the examples described below can be appropriately changed as long as they are within the gist of the present invention. Accordingly, the scope of the present invention should not be limitatively interpreted by the specific examples described below. Examples

### (Preparation of microneedle array)

An aqueous solution obtained by mixing 34 mg/mL of hydroxyethyl starch (HES) (Fresenius Kabi, average molecular weight of 70000) and 8.5 mg/mL of human serum albumin (HSA) (Wako Pure Chemical Industries, Ltd.) (drug model) was prepared, and a mold having recesses in a conical shape was filled with the aqueous solution and then dried. A stainless steel (SUS 304) frame (thickness of 0.1 to 0.5 mm, diameter of 12 mm or 15 mm) was placed on the mold filled with the aqueous solution. Here, a water-soluble polymer-dissolved solution (any one of hydroxyethyl starch or dextran 70) whose concentration and liquid amount were adjusted and which did not contain a drug was directly applied, and the needle-like recesses were filled with the water-soluble polymer-dissolved solution which did not contain a drug and dried (temperature of 23°C, relative humidity of 45%). After the needle-like recesses were dried, a microneedle array having needle tips and needle bases formed of different water-soluble polymers was obtained by being taken out from the frame and the mold.

In the above-described operation, in a case where the microneedle array in a drying step is in a semi-dry state (for example, a paste state in which the shape of the solution changes even though the solution does not have fluidity) and the frame (preferably a stainless steel frame) and the mold are curved together, the frame and the mold are dried in a state of having an optional curved shape, and thus a sheet having an optional deformed shape is molded. In Examples 1, 2, and 4, each microneedle array was prepared according to the present technique. Further, the deformation height was controlled by controlling the dropping amount and the thickness of the frame. In Examples 3, 5 to 11, and Comparative Examples 1 to 5, each microneedle array was prepared according to the present technique.

The microneedle prepared in the above-described manner includes frustums and needles. The height of a needle is approximately 600 µm and the width of a base portion is approximately 270 µm. The frustum has a truncated cone structure, and the height of the frustum is approximately 130 µm, the diameter of the upper bottom surface of the frustum is approximately 270 µm, and the diameter of the lower bottom surface of the frustum is approximately 460 µm. The number of needles is 100, the interval between needles is approximately 1 mm, and the needles are arranged in a square shape.

### (Measurement of average film thickness of end portion of microneedle array)

The microneedle array was placed on a plane and observed using a microscope (VHX-5500, manufactured by KEYENCE CORPORATION) at an angle of 90° horizontally. The film thickness between two points of end portions on a straight line acquired by obtaining the end portions farthest from the center of the sheet and connecting the center of the sheet and the end portions farthest from the center thereof was measured. The average film thickness of the end portion was measured based on the measurement results, and the measurement results are collectively listed in Table 1.

### (Measurement of average film thickness of central portion of microneedle array)

The microneedle array was cut so as to pass through the central portion of the sheet, the exposed cut cross section of the central portion was observed using a microscope (VHX-5500, manufactured by KEYENCE CORPORATION) at an angle of 90° horizontally, measuring the film thickness (the film thickness of three sites in total) of the central portion and adjacent two sites separated from the central portion by a distance of 1 mm, and the average film thickness thereof was calculated as the average film thickness of the central portion. The measurement results are collectively listed in Table 1.

### (Measurement of deformation height of microneedle array)

The microneedle array was placed on the target to be punctured and observed using a microscope (VHX-5500, manufactured by KEYENCE CORPORATION) at an angle of 90° horizontally. The absolute value of the deformation height was measured by measuring the height from the target to a point positioned at the most distant place. The absolute value of the deformation height was calculated based on the measurement results and the measurement results are collectively listed in Table 2.

### (Evaluation (i) of puncture into pig skin)

After the subcutaneous fat of the skin collected from the back of a pig was removed, the skin was placed on a cushion simulating the human skin. The prepared microneedle array was placed on the pig skin such that the needles were in contact with the pig skin, and the microneedle array was punctured into the pig skin at an energy of 0.5 J using an applicator in an environment of a temperature of 25°C and a relative humidity of 60%. The microneedle array remaining on the pig skin was observed immediately after the microneedle array was punctured into the pig skin, and a state of damage (breakage) to the sheet was evaluated. A case where the sheet was not damaged (not broken) was evaluated as A and a case where the sheet was damaged (broken) was evaluated as B. The results are listed in Table 1.

**[Table 1]**

| | Concentration | Liquid height | Thickness of frame | Material for sheet | Average film thickness of end portion/average film thickness of central portion | Average film thickness of central portion | Evaluation of puncture (i) |
|---|---|---|---|---|---|---|---|
| Example 1 | 40% | 730 µm | 0.1 mm | Dextran 70 | 0.3 times | 292 µm | A |
| Example 2 | 40% | 575 µm | 0.1 mm | Dextran 70 | 0.5 times | 230 µm | A |
| Example 3 | 40% | 513 µm | 0.1 mm | Dextran 70 | 0.5 times | 205 µm | A |
| Example 4 | 40% | 518 µm | 0.1 mm | Dextran 70 | 0.6 times | 207 µm | A |
| Example 5 | 40% | 463 µm | 0.1 mm | Dextran 70 | 0.7 times | 185 µm | A |
| Example 6 | 40% | 258 µm | 0.1 mm | Dextran 70 | 0.9 times | 103 µm | A |
| Example 7 | 40% | 765 µm | 0.3 mm | Dextran 70 | 0.9 times | 306 µm | A |
| Example 8 | 40% | 758 µm | 0.1 mm | Hydroxyethyl starch | 0.3 times | 303 µm | A |
| Example 9 | 40% | 505 µm | 0.1 mm | Hydroxyethyl starch | 0.5 times | 202 µm | A |
| Example 10 | 40% | 473 µm | 0.1 mm | Hydroxyethyl starch | 0.7 times | 189 µm | A |
| Example 11 | 40% | 265 µm | 0.1 mm | Hydroxyethyl starch | 0.9 times | 106 µm | A |
| Comparative Example 1 | 40% | 253 µm | 0.4 mm | Dextran 70 | 4 times | 101 µm | B |
| Comparative Example 2 | 40% | 190 µm | 0.4 mm | Dextran 70 | 5 times | 76 µm | B |
| Comparative Example 3 | 40% | 750 µm | 0.5 mm | Dextran 70 | 1.5 times | 300 µm | B |
| Comparative Example 4 | 40% | 235 µm | 0.4 mm | Hydroxyethyl starch | 4 times | 94 µm | B |
| Comparative Example 5 | 40% | 110 µm | 0.5 mm | Hydroxyethyl starch | 10 times | 44 µm | B |

### (Evaluation (ii) of puncture into pig skin)

Next, evaluation (ii) of puncture was performed in an environment of a temperature of 15°C and a relative humidity of 20% using each microneedle array of Examples 1 to 11 in which the sheet was not damaged from the evaluation (i) of puncture. The evaluation was performed under the same conditions as in the evaluation (i) of puncture except for the puncture environment. The evaluation was performed using the same technique as in the evaluation (i) of puncture, and a case where the sheet was not damaged (not broken) was evaluated as A and a case where the sheet was damaged (broken) was evaluated as B. The results are listed in Table 2. Microneedle arrays according to the invention are those having an absolute value of a deformation height of the curved surface in a range of 1 µm to 300 µm, thus, examples 13 and 18.

**[Table 2]**

| | Absolute value of deformation height of curved surface shape | Deformation direction | Film thickness distribution of microneedles | Evaluation (ii) of puncture |
|---|---|---|---|---|
| Example 12 | 721 µm | Projection on top | Similar to Example 1 | B |
| Example 13 | 221 µm | Projection on top | Similar to Example 2 | A |
| Example 14 | 435 µm | Projection on bottom | Similar to Example 3 | B |
| Example 15 | 733 µm | Projection on top | Similar to Example 4 | B |
| Example 16 | 429 µm | Projection on bottom | Similar to Example 5 | B |
| Example 17 | 436 µm | Projection on bottom | Similar to Example 6 | B |
| Example 18 | 278 µm | Projection on bottom | E Similar to xample 7 | A |
| Example 19 | 1855 µm | Projection on bottom | Similar to Example 8 | B |
| Example 20 | 1235 µm | Projection on bottom | Similar to Example 9 | B |
| Example 21 | 1344 µm | Projection on bottom | Similar to Example 10 | B |
| Example 22 | 1311 µm | Projection on bottom | Similar to Example 11 | B |

As listed in Table 1, breakage did not occur at the time of puncture in all microneedle array groups in which the average film thickness of the end portion was smaller than the average film thickness of the central portion. On the contrary, breakage occurred in the microneedle array groups in which the average film thickness of the end portion was larger than the average film thickness of the central portion. The reason for this is assumed that, since the sheet was enclosed by the end portion in a wall surface shape, the internal stress occurring due to the deformation at the time of puncture was not able to escape to the outside, and thus the sheet was broken due to the concentration of the stress inside the sheet.

As listed in Table 2, in a low humidity environment in which the sheet was easily damaged, there was a tendency that the sheet with a smaller deformation height was not damaged. It is assumed that the sheet was likely to be damaged because the deformation amount at the time of following the target to be punctured was increased in the microneedle array having a high deformation height of the sheet with respect to the target to be punctured. This suggests that the followability between the target to be punctured and the sheet of the microneedle array is a factor of suppressing the damage.

As described above, it is necessary for the microneedle array to have appropriate strength and flexibility for administration of a drug into the skin through reliable puncture. At this time, it is advantageous that the internal stress in the sheet easily escapes. In a case of assuming reliable puncture into the human skin, the film thickness distribution needs to be made under a condition in which the average film thickness of the end portion is smaller than the average film thickness of the central portion.

### Explanation of References

1: microneedle array
2: microneedle array
110: microneedle
112: needle
112A: first layer of needle
112B: second layer of needle
112C: needle third layer
116: sheet
120: layer
122: layer
320: frame
322: opening portion
W: diameter (width)
H: height
H1: height
H2: height
H3: height
T: height (thickness)
11: original plate
12: shaped portion
13: mold
15: needle-like recess
15A: inlet portion
15B: tip recess
15C: air vent hole
D: diameter (diameter)
18: mold complex
19: air permeating sheet
20: base
22: water-soluble polymer-dissolved solution
24: water-soluble polymer-dissolved solution
30: tank
32: pipe
34: nozzle
34A: lip portion
34B: opening portion
36: liquid supply device
P1: pressing pressure
P2: pressing force
P3: pressing force
40: base material
61: X-axis drive unit
62: Z-axis drive unit
63: nozzle
64: liquid supply device
65: suction stand
66: laser displacement meter
67: load cell
68: control mechanism
69: mold

## Claims

1. A microneedle array comprising:
a sheet; and
a plurality of needles which are present on an upper surface of the sheet,
wherein the needles contain a water-soluble polymer,
the sheet contains a water-soluble polymer,
the sheet has a central portion, which is a region where the plurality of needles are formed, and an end portion, and
an average film thickness of the end portion is smaller than an average film thickness of the central portion,
wherein the sheet has a curved surface shape, and
an absolute value of a deformation height of the curved surface is in a range of 1 µm to 300 µm.

2. The microneedle array according to claim 1,
wherein the average film thickness of the end portion is 0.3 to 0.9 times the average film thickness of the central portion.

3. The microneedle array according to claim 1 or 2,
wherein the average film thickness of the central portion is in a range of 100 µm to 300 µm.

4. The microneedle array according to any one of claims 1 to 3,
wherein the needles contain a drug.

5. The microneedle array according to any one of claims 1 to 4,
wherein a drug is one or more selected from a peptide hormone, a vaccine, and an adjuvant.

6. The microneedle array according to any one of claims 1 to 5,
wherein the water-soluble polymer contained in the sheet contains dextran or hydroxyethyl starch.

7. The microneedle array according to any one of claims 1 to 6,
wherein the water-soluble polymer of a needle base and the water-soluble polymer of the sheet are the same as each other.

## Patentansprüche

1. Mikronadel-Array, aufweisend:
ein Flachmaterial; und
eine Mehrzahl von Nadeln, die an einer oberen Oberfläche des Flachmaterials vorhanden sind,
wobei die Nadeln ein wasserlösliches Polymer enthalten,
das Flachmaterial ein wasserlösliches Polymer enthält,
das Flachmaterial einen mittigen Bereich, der eine Region ist, in der die Mehrzahl von Nadeln ausgebildet sind, und einen Endbereich hat, und
die mittlere Filmdicke des Endbereichs kleiner ist als die mittlere Filmdicke des mittigen Bereichs,
wobei das Flachmaterial eine gewölbte Oberflächenform hat, und
der Absolutwert der Verformungshöhe der gewölbten Oberfläche in einem Bereich von 1 µm bis 300 µm ist.

2. Mikronadel-Array nach Anspruch 1,
wobei die mittlere Filmdicke des Endbereichs das 0,3- bis 0,9-fache der mittleren Filmdicke des mittleren Bereichs ist.

3. Mikronadel-Array nach Anspruch 1 oder 2,
wobei die mittlere Filmdicke des mittigen Bereichs in einem Bereich von 100 µm bis 300 µm ist.

4. Mikronadel-Array nach einem der Ansprüche 1 bis 3,
wobei die Nadeln ein Arzneimittel enthalten.

5. Mikronadel-Array nach einem der Ansprüche 1 bis 4,
wobei das Arzneimittel eines oder mehrere der Arzneimittel ist, die ausgewählt sind aus einem Peptidhormon, einem Impfstoff und einem Hilfsstoff.

6. Mikronadel-Array nach einem der Ansprüche 1 bis 5,
wobei das wasserlösliche Polymer, das in dem Flachmaterial enthalten ist, Dextran oder Hydroxyethyl-Stärke enthält.

7. Mikronadel-Array nach einem der Ansprüche 1 bis 6,
wobei das wasserlösliche Polymer einer Nadelbasis und das wasserlösliche Polymer des Flachmaterials einander gleich sind.

## Revendications

1. Réseau de micro-aiguilles, comprenant :
une feuille, et
une pluralité d'aiguilles, lesquelles sont présentes sur une surface supérieure de la feuille,
dans lequel les aiguilles contiennent un polymère soluble dans l'eau ;
la feuille contient un polymère soluble dans l'eau ;
la feuille présente une portion centrale, laquelle est une région où la pluralité d'aiguilles sont formées, et une portion d'extrémité, et
une épaisseur de film moyenne de la portion d'extrémité est inférieure à une épaisseur de film moyenne de la portion centrale :
dans lequel la feuille présente une forme de surface incurvée, et
une valeur absolue d'une hauteur de déformation de la surface incurvée est comprise dans une plage allant de 1 µm à 300 µm.

2. Réseau de micro-aiguilles selon la revendication 1,
dans lequel l'épaisseur de film moyenne de la portion d'extrémité s'étend de 0,3 à 0,9 fois l'épaisseur de film moyenne de la portion centrale.

3. Réseau de micro-aiguilles selon la revendication 1 ou 2,
dans lequel l'épaisseur de film moyenne de la portion centrale est comprise dans une plage allant de 100 µm à 300 µm.

4. Réseau de micro-aiguilles selon l'une quelconque des revendications 1 à 3,
dans lequel les aiguilles comprennent un médicament.

5. Réseau de micro-aiguilles selon l'une quelconque des revendications 1 à 4,
dans lequel un médicament est un ou plusieurs éléments sélectionnés parmi une hormone peptidique, un vaccin, et un adjuvant.

6. Réseau de micro-aiguilles selon l'une quelconque des revendications 1 à 5,
dans lequel le polymère soluble dans l'eau contenu dans la feuille contient de la dextrane ou de l'amidon hydroxyéthylé.

7. Réseau de micro-aiguilles selon l'une quelconque des revendications 1 à 6,
dans lequel le polymère soluble dans l'eau d'une base d'aiguille et le polymère soluble dans l'eau de la feuille sont identiques entre eux.
